(19) Europäisches Patentamt
European Patent Office
Office européen des brevets

(11) EP 3 345 916 B1

(12) **EUROPEAN PATENT SPECIFICATION**

(45) Date of publication and mention of the grant of the patent:
**23.03.2022 Bulletin 2022/12**

(21) Application number: **17749894.6**

(22) Date of filing: **09.02.2017**

(51) International Patent Classification (IPC):
***A61K 31/58*** *(2006.01)* ***A61P 31/12*** *(2006.01)*
***A61P 31/14*** *(2006.01)* ***A61P 11/00*** *(2006.01)*
***A61P 27/16*** *(2006.01)* ***A61K 31/4355*** *(2006.01)*

(52) Cooperative Patent Classification (CPC):
**A61K 31/4355; A61P 31/14; C07J 71/0005**

(86) International application number:
**PCT/CN2017/073133**

(87) International publication number:
**WO 2017/137003 (17.08.2017 Gazette 2017/33)**

(54) **COMPOUND FOR TREATING RESPIRATORY SYNCYTIAL VIRUS INFECTION AND PREPARATION METHOD AND USE THEREOF**

VERBINDUNG ZUR BEHANDLUNG DES RESPIRATORISCHEN SYNZYTIALVIRUS SOWIE HERSTELLUNGSVERFAHREN UND VERWENDUNG DAVON

COMPOSÉ DESTINÉ AU TRAITEMENT DE L'INFECTION PAR LE VIRUS SYNCYTIAL RESPIRATOIRE ET SON PROCÉDÉ DE PRÉPARATION ET D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2016 CN 201610084979**

(43) Date of publication of application:
**11.07.2018 Bulletin 2018/28**

(73) Proprietor: **Shanghai Medusa Biotechnology Co., Ltd.**
**Shanghai 201906 (CN)**

(72) Inventors:
- **ALTMEYER, Ralf**
  **Jinan**
  **Shandong 250100 (CN)**
- **CAO, Jingjing**
  **Jinan**
  **Shandong 250100 (CN)**

(74) Representative: **Niburska, Danuta**
**Kancelaria Patentowa**
**Al. 3 Maja 68 B**
**76-200 Slupsk (PL)**

(56) References cited:
**WO-A2-2006/026430** **WO-A2-2008/083248**
**CN-A- 105 232 562** **CN-A- 105 232 562**

- **RUIXIN ZHU ET AL: "Investigations on Inhibitors of Hedgehog Signal Pathway: A Quantitative Structure-Activity Relationship Study", INTERNATIONAL JOURNAL OF MOLECULAR SCIENCES, vol. 12, no. 5, 11 May 2011 (2011-05-11), pages 3018-3033, XP055486874, DOI: 10.3390/ijms12053018**
- **CHEN J K ET AL: "Small molecule modulation of Smoothened activity", PROCEEDINGS OF THE NATIONAL ACADEMY OF SCIENCES, NATIONAL ACADEMY OF SCIENCES, US, vol. 99, no. 22, 29 October 2002 (2002-10-29), pages 14071-14076, XP002251705, ISSN: 0027-8424, DOI: 10.1073/PNAS.182542899**
- **BAILLY, B. ET AL.: 'Targeting human respiratory syncytial virus transcription antitermination factor M2-1 to inhibit in vivo viral replication' SCIENTIFIC REPORTS no. 6, 19 May 2016, pages 1 - 11, XP055407561**
- **ZHOU, JIANXIA ET AL.: 'Advances in the Reasearch of a Steroidal Alkaloid Cyclopamine' CHINESE JOURNAL OF NATURAL MEDICINES vol. 4, no. 6, 30 November 2006, pages 468 - 472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 345 916 B1

## Description

The invention relates to biomedical technology, and more particularly to a compound for treating respiratory syncytial virus infection, a preparation method and use thereof.

Bronchiolitis is a severe lower-respiratory tract infectious disease primarily caused by members of the *Paramyxoviridae* family. Human respiratory syncytial virus (hRSV) is the main cause of morbidity in children less than 2 years of age as well as the elderly, the immuno-compromised, and the transplant patients. By now, there are neither vaccines nor efficacious approved drugs to prevent or treat hRSV infection. The immuno-prophylactic antibody palivizumab is approved for high-risk patients, such as premature babies and infants suffering from underlying diseases. The broad-spectrum and small molecule antiviral ribavirin is available to treat infection, but it has considerable side-effects and limited efficacy. During the past decade, a number of drug candidates targeting hRSV entry or replication steps have been advanced to pre-clinical or clinical development.

The hRSV genomic RNA (vRNA) is packaged by the viral nucleoprotein (N) at all times, forming a N:RNA complex, called nucleocapsid. This ribonucleoprotein complex is used as a template for mRNA transcription and genomic or antigenomic RNA replication by the RNA-dependent RNA polymerase (RdRp), which is composed of two major viral proteins: the phosphoprotein P and the large polymerase L. The polymerase L is combined with the nucleoprotein N, and the polymerase L is bound to vRNA. In this process, the phosphoprotein is an essential co-factor of the L polymerase. Two co-factors, M2-1 and M2-2, are required for the RdRp to process RNA efficiently during the viral cycle. M2-1 is a tetrameric transcription processivity factor that binds in a competitive manner to RNA and P via its core domain. M2-1 functions as an anti-terminator of transcription that prevents premature termination of transcription both intra- and inter-genetically. Although *in vitro* experiments have shown that M2-1 binds preferentially to positive-sense viral gene end (GE) and poly-A sequences, the exact mechanisms by which M2-1 improves transcription efficiency is not fully understood.

Recently, cyclopamine (CPM) and jervine were identified as highly potent and selective inhibitors of hRSV replication *in vitro.* CPM is a well-known antagonist of the smoothened protein (Smo) receptor, a 7-transmembrane receptor of the Sonic hedgehog signaling pathway (Shhp). The Shhp is involved in embryonic development, cell differentiation, and tumorigenesis. Cyclopamine is a naturally occurring compound that belongs to the group of steroidal jerveratrum alkaloids. It is a teratogen isolated from the corn lily (Veratrum californicum) that causes usually fatal birth defects. It can prevent the fetal brain from dividing into two lobes (holoprosencephaly) and causes the development of a single eye (cyclopia). These defects are caused by inhibiting the hedgehog signaling pathway (Hh).

The teratogenic properties of cyclopamine largely preclude its development as a pediatric drug for the treatment of hRSV infections. These properties could lead to serious adverse events in treated patients.

In order to overcome the teratogenic property of CPM, and to fill the gap in clinical RSV infection treatment, especially for use in pediatrics, the present invention provides a compound for treating RSV infection and its preparation method and use thereof. The compound inhibits RSV replication, and does not inhibit Hedgehog signaling pathway. Therefore, the compound is a safe and effective anti-RSV drug for clinic application, relieving of teratogenicity.

To achieve the above objective, in accordance with one embodiment of the invention, there is provided a compound for treating RSV infection that has the property of inhibiting RSV replication and that does not inhibit Hedgehog signaling pathways.

In a class of this embodiment, the compound is a novel chemical compound S1-2 or S1-4 having the property of inhibiting the replication of respiratory syncytial virus without inhibiting the Hedgehog signaling pathway.

The Chinese patent CN 105232562 recites use of cyclopamine to suppress replication of respiratory syncytial virus.

As a comparative example, CPM has a structure of formula 1.

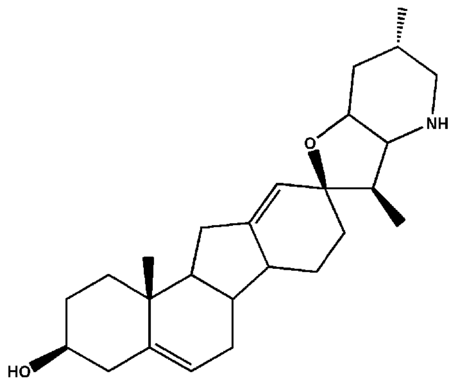

1

As another comparative example, a compound has a structure of formula I,

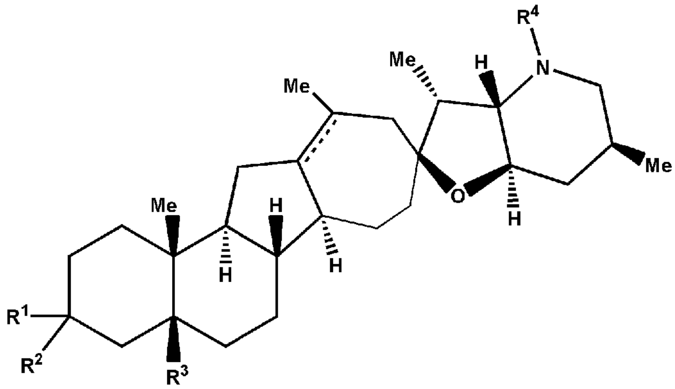

I

in which, $R_1$ is H, alkyl, amino, sulfinyl amino, sulfonyl amino, - $OC(O)R_5$, $-N(R_5)C(O)R_5$, or glycosyl;

$R_2$ is H, alkyl, alkenyl, alkynyl, aryl, cycloalkyl, cyano, or heterocyclic alkyl; or $R_1$ and $R_2$ together form =O, =S, =N(R), $=N(NR_2)$ or $=C(R)_2$;

$R_3$ is H, alkyl, alkenyl, or alkynyl;

$R_4$ is H, alkyl, alkenyl, alkynyl, aryl, cycloalkyl, heterocyclic alkyl, aralkyl, heterocyclic aryl, heterocyclic aralkyl, halogenated alkyl, $-OR_5$,$-C(O)R_5$, $-CO_2R_5$, $-SO_2R_5$, $-C(O)N(R_5)(R_5)$, $-[C(R)_2]_q-R_5$, $-[(W-N(R)C(O)]_qR_5$,$-[(W-C(O)]_qR_5$, $-[(W)-C(O)O]_qR_5$, $-[(W)-OC(O)]_qR_5$, $-[(W)-SO_2]_qR_5$, $-[(W)-N(R_5)SO_2]_qR_5$, $-[(W)-C(O)N(R_5)]_qR_5$, $-[(W)-O]_qR_5$, $-[(W)-N(R)]_qR_5$, $-W-NR_5^{3+}X^-$, or $-[(W)-S]_qR_5$;

in which, W is divalent alkyl independently;

R is H or alkyl independently;

q is 1, 2, 3, 4, 5, or 6 independently;

X- is halogen;

$R_5$ is H, alkyl, alkenyl, alkynyl, aryl, cycloalkyl, heterocyclic alkyl, aralkyl, heterocyclic aryl, heterocyclic aralkyl, or $-[C(R)_2]_p-R_6$; p is an integer of 0-6; or any two $R_5$ groups on the same substituent group form a 4-8 membered ring containing 0-3 heteroatoms, and the heteroatoms are selected from the group consisting of N,O, S and P; $R_6$ is independently hydroxyl,$-N(R)COR$, $-N(R)C(O)OR$, $-N(R)SO_2(R)$, $-C(O)N(R)_2$, $-OC(O)N(R)(R)$,$-SO_2N(R)(R)$,

-N(R)(R), -COOR, -C(O)N(OH)(R), -OS(O)$_2$OR, -S(O)$_2$OR,-OP(O)(OR)(OR), -NP(O)(OR)(OR) or -P(O)(OR)(OR). In a class of this embodiment, $R_1$ is not hydroxyl or glycosyl when $R_2$, $R_3$ and $R_4$ are H. In another class of this embodiment, $R_1$ and $R_2$ are not C=O when $R_4$ is hydroxyl.

In another comparative example, the compound has a structure of formula II,

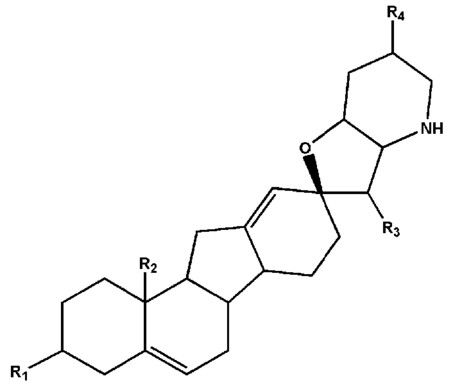

(II)

in which, $R_1$ is H, OH, alkyl, amino, sulfonyl amino, sulfinyl amino,-OC(O)$R_5$, -N($R_5$)C(O)$R_5$, or glycosyl;

$R_2$ is H, alkyl, alkenyl, alkynyl, aryl, cycloalkyl, cyano, or heterocyclic alkyl;

$R_3$ is H, alkyl, alkenyl, alkynyl;

$R_4$ is H, alkyl, alkenyl, alkynyl, aryl, cycloalkyl, heterocyclic alkyl, aralkyl, heterocyclic aryl, heterocyclic aralkyl, halogenated alkyl, -O$R_5$,-C(O)$R_5$, -CO$_2R_5$, -SO$_2R_5$, -C(O)N($R_5$)($R_5$), -[C(R)$_2$]$_q$-$R_5$, -[(W)-N(R)C(O)]q$R_5$,-[(W-C(O)]$_q R_5$, -[(W)-C(O)O]$_q R_5$, -[(W)-OC(O)]$_q R_5$, -[(W)-SO$_2$]q$R_5$, -[(W)-N($R_5$)SO$_2$]$_q R_5$, -[(W)-C(O)N($R_5$)]$_q R_5$, -[(W)-O]$_q R_5$, -[(W)-N(R)]$_q R_5$, -W-N$R_5^{3+}$X$^-$, or -[(W)-S]$_q R_5$;

In which, W is divalent alkyl independently;

R is H or alkyl independently;

q is 1, 2, 3, 4, 5, or 6 independently;

X- is halogen;

$R_5$ is H, alkyl, alkenyl, alkynyl, aryl, cycloalkyl, heterocyclic alkyl, aralkyl, heterocyclic aryl, heterocyclic aralkyl, or -[C(R)$_2$]p-$R_6$; in which p is an integer of 0-6; or any two $R_5$ groups on the same substituent group form a 4-8 membered ring containing 0-3 heteroatoms, and the heteroatoms are selected from the group consisting of N,O, S and P; $R_6$ is independently hydroxyl,-N(R)COR, -N(R)C(O)OR, -N(R)SO$_2$(R), -C(O)N(R)$_2$, -OC(O)N(R)(R),-SO$_2$N(R)(R), -N(R)(R), -COOR, -C(O)N(OH)(R), -OS(O)$_2$OR, -S(O)$_2$OR,-OP(O)(OR)(OR), -NP(O)(OR)(OR), or -P(O)(OR)(OR);

In a class of this comparative example, in formula II, $R_1$ is OH, H, or substituted or unsubstituted $C_1$-$C_3$ alkyl group;

$R_2$ is substituted or unsubstituted $C_1$-$C_3$ alkyl, alkenyl, alkynyl;

$R_3$ is substituted or unsubstituted $C_1$-$C_3$ alkyl, alkenyl, alkynyl;

$R_4$ is substituted or unsubstituted $C_1$-$C_3$ alkyl, alkenyl, alkynyl;

The term "substituted" means that, one or more H atoms in the hydrocarbyl were replaced by $C_1$-$C_{10}$ alkyl, $C_3$-$C_{10}$ cycloalkyl, $C_1$-$C_{10}$ alkoxy, hydroxyl, carboxyl, $C_1$-$C_{10}$ carbonyl, $C_1$-$C_{10}$ acylamino, $C_2$-$C_{10}$ ester group, $C_6$-$C_{30}$ aryl,

halogen atom, cyano, or thioether group;

Preferably, $R_1$ is OH, $R_2$ is methyl, $R_3$ is methyl, and $R_4$ is methyl.

The compound S1-2 of formula III is obtained by replacing $R_1$ and $R_4$ in formula II.
The compound S1-4 of formula IV is obtained by replacing $R_1$ and $R_4$ in formula II.
The difference between S1-2 and S1-4 is that $R_1$ in formula II is replaced with different substituent groups.

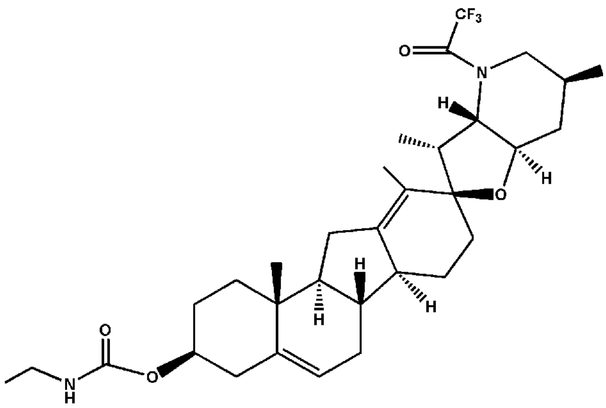

Series 1-2

(III)

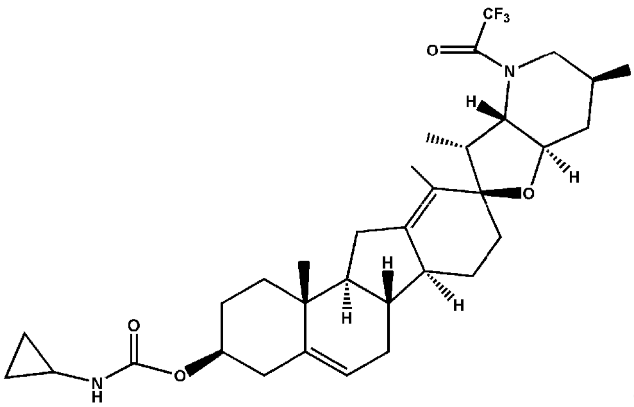

Series 1-4

(IV)

In accordance with another embodiment of the invention, there is provided a method for preparing a compound for treating respiratory syncytial virus infection. The method comprises synthesizing a plurality of compounds, and screening the compounds by two parallel *in vitro* tests. The *in vitro* tests include: (1) based on cell experiments, measuring the replication ability of respiratory syncytial virus in excitable cells, and (2) based on cell experiments, measuring the inhibition of sonic hedgehog pathway of a molecule, the inhibition effect being interference level on SAG-induced and Smo-dependent gene transcription.
In a class of this embodiment, the method comprises:

1) synthesizing the compounds; and

2) screening the compounds.

In a class of this embodiment, synthesizing the compounds comprises two sub-steps:

1) preparation of intermediates; and
2) preparation of the compounds.

In a class of this embodiment, screening the compounds comprises four sub-steps:

1) testing the inhibitory effect of the compounds on the replication of respiratory syncytial virus;
2) testing the inhibitory effect of the compounds on the Hh signaling pathway;
3) calculating respiratory syncytial virus replication and Hh activity inhibition coefficient; and
4) screening the compounds.

In a class of this embodiment, the preparation of intermediates comprises: firstly, dissolving cyclopamine and triethylamine in dry dichloromethane (DCM); secondly, adding trifluoroacetic acid anhydride dissolved in DCM into the solution under 0°C; thirdly, heating the reaction mixture slowly to a temperature of 15°C and stirring for 16 h; fourthly, confirming the consumption of the initial raw materials by liquid chromatography - mass spectrometry; fifthly, concentrating the reaction mixture and diluting the residues with methanol; sixthly, heating the obtained suspension to 70°C and holding for 1 h; seventhly, cooling the reaction mixture to 15°C; and finally, filtrating to obtain white solid intermediates.

In a class of this embodiment, the preparation of the compounds comprises: firstly, dissolving the intermediates and triethylamine in dry DCM, cooling in ice; secondly, adding isocyanato ethane dissolved in DCM into the solution; thirdly, stirring the reaction mixture for 48 h at 45°C; fourthly, confirming that the initial raw materials are consumed completely by TLC; fifthly, concentrating the reaction mixture in vacuum; sixthly, cleaning the residues with methanol; and finally, filtrating to obtain white solid compounds.

In a class of this embodiment, testing the inhibitory effect of the compounds on the replication of RSV comprises the following steps:

firstly, human respiratory syncytial virus Long strain (ATCC NO. VR-26) and HEp-2 cells (ATCC NO. CCL-23) are used in the infection assays;

secondly, cells are maintained in DMEM, supplemented with penicillin/streptomycin and 10% fetal bovine serum (FBS), and incubated at 37°C, 5% $CO_2$;

thirdly, virus is passaged in HEp-2 cells under the same conditions, except that a concentration of FBS is 2%;

fourthly, virus stocks are prepared by infecting confluent HEp-2 cells at a low multiplicity of infection (MOI) of 0.1 for two to three days; cells and virus-containing supernatant are then subjected to a single freeze/thaw cycle at -80°C to release cell-bound virus;

fifthly, the sample is clarified by centrifugation at a speed of 2000xg for 10 min at 4°C, the supernatant is homogenized, aliquoted and stored at - 20°C;

sixthly, the anti-hRSV potency of the compounds is evaluated by focus reduction assay, titer calculation is based on the wells with 50 to 100 focus forming; compounds are tested either during virus adsorption process with compounds treatment for 1 h at 4°C to investigate the effect of the compounds on viral entry process, or during post-adsorption process with compounds treatment for 72 h at 37°C to investigate the effect of the compounds on viral propagation and replication processes, or during all stages of infection; the concentration of compounds resulting in 50% inhibition ($IC_{50}$) of virus replication is determined by focus counting (viral entry process) or focus size measurement (viral propagation process) and by using non-linear regression analysis through GraphPad Prism software; and

a CellTiter-Glo Luminescent Cell Viability Assay is carried following the manufacturer's instruction under conditions of the focus reduction assay to assess the cytotoxicity of the compounds.

In a class of this embodiment, testing the inhibitory effect of the compounds on Hh signaling pathway comprises the following process:

firstly, NIH3T3 cells are seeded in a 6-well plate, or a 12-well plate one day in advance, cultivated with DMEM comprising 5% FBS and no antibiotics so as to ensure 90-95% confluence at the time of transfection;

secondly, diluted plasmids are mixed with Lipofectamine 2000, cultivated and are added to each well containing cells and medium, then the plate is incubated at 37°C in a $CO_2$ incubator for 48 h;

thirdly, after 48 h transfection, SAG is added until a final concentration thereof is 0.5 μM, and the compounds of

various concentrations are added;

fourthly, at the end of the incubation, cells are washed, lysed and the lysate is transferred to a 96-well white plate; and

fifthly, LAR II reagent (from Promega, Dual-luciferase reporter assay system) is added, and the data for the samples are read in a luminescence reader; the compounds in the RSV inhibition assay are tested in the Hh signaling pathway assay, in which the inhibition effect of the compounds on Smo-dependent SAG induced gene expression is tested.

In the Hh signaling pathway inhibition assay, the compound is selected from those used in the RSV inhibition assay and those show effective inhibition on RSV replication. In the Hh signaling pathway inhibition assay, the inhibition effect of the compound on SAG-induced and Smo-dependent gene expression is analyzed.

To assess the difference between Hh signaling pathway inhibition effect and RSV replication inhibition effect of the compound (cpd), a coefficient of inhibition (ICrh) is defined as follows:

$$ICrh = \frac{cpd\left(\frac{\%RSV\,inhib}{\%Hh\,inhib}\right)}{CPM\left(\frac{\%RSV\,inhib}{\%Hh\,inhib}\right)}$$

In the formula, the denominator is the inhibition ratio of CPM, i.e., the ratio of CPM's inhibition effect on RSV replication to CPM's inhibition effect on Hh signaling pathway. The numerator is the inhibition ratio of the compound. According to the definition of the coefficient of inhibition (ICrh), if the compound tested is CPM, the coefficient of inhibition is 1. If a compound has an inhibition coefficient lower than 1, it has an unfavorable inhibition profile, eg., the anti-RSV activity is low, while the inhibition on the Hh signaling pathway is still strong. If the compound has an inhibition coefficient higher than 1, it has a favorable inhibition profile.

In a class of this embodiment, the substep of screening the compounds comprises selecting the compounds with an ICrh value larger than 1, and excluding the compounds with an ICrh value smaller than 1. Furthermore, the compounds with higher ICrh values are selected preferentially.

In accordance with another embodiment of the invention, the compounds are for use in the treatment of diseases caused by respiratory infection. Briefly, the disease is respiratory virus infection, respiratory syncytial virus infection, capillary bronchitis caused by respiratory syncytial virus, pneumonia caused by respiratory syncytial virus, or tympanitis caused by respiratory syncytial virus. Furthermore, the compounds never cause side effects on patients, such as fetal anomaly.

The advantages and beneficial effects of the invention are as follows:

1) The compounds in this invention are effective in the treatment of the following diseases: respiratory virus infection, respiratory syncytial virus (RSV) infection, and capillary bronchitis / pneumonia / tympanitis caused by respiratory syncytial virus.
2) the compounds in this invention never cause side effects on patients, such as fetal anomaly.
3) the preparation method in this invention can be used to synthesize various compounds for use in RSV infection treatment, and the compounds have no side effects.
4) The preparation methods in the invention is easy, and has wide material source, low production cost, and high economic value.
5)The compounds in the invention fill the gap in medicines for the treatment of RSV infection, especially for use in pediatrics, and has great value.

The invention will be further described with the following figures and examples.

FIG. 1 is the flow diagram of the preparation method in this invention.
FIG. 2 is the flow diagram of the preparation method for the intermediates in the invention.
FIG. 3 is the flow diagram of the preparation method for the compounds in the invention.
FIG. 4 shows the inhibition effect of the compounds in the invention on RSV replication.

As shown in FIG. 4, the compounds were tested at two concentrations (2 μM, 8 μM) in the RSV replication assay. For the control group (with no test compounds), the replication ratio is 100%. For the CPM group, the replication ratio is 0%. Compared with the control group, the compounds S2-6, and C2-2 to C2-4 are able to inhibit RSV replication to certain extent.

FIG. 5 shows the inhibition effect of the compounds in the invention on Smo induced gene expression.

As shown in FIG. 5, the various compounds were tested at 10 μM in the hedgehog (Hh) signaling pathway assay. For the control group (without any test compounds, but contains SAG), the gene expression ratio is 100%. The gene expression ratio is significantly reduced by 56% in the presence of CPM. Gene expression inhibition ratios are not significant for the compounds S1-2 and S1-4, and are 36% and 5% respectively. These data show that the anti-RSV activity of the steroidal alkaloid molecules derived from CPM is independent from their inhibitory activity on the Smo-dependent Hh pathway.

FIG. 6 shows the inhibition of coefficient (ICrh) of CPM and the compounds of the invention.

Example 1

The preparation process of the compound S1-2, by referring to FIGS. 1, 2, and 3, is as follows:

1) Preparation of intermediate. Cyclopamine (498.06 mg, 1.21 mmol, 1.00 eq) and triethylamine ($Et_3N$) (489.76 mg, 4.84 mmol, 4.00 eq) were dissolved in dry DCM (4 ml). Then trifluoracetic anhydride (TFAA) (762.41 mg, 3.63 mmol, 3.00 eq) dissolved in 1 ml DCM was added at 0°C. The reaction mixture was slowly heated to 15°C, and stirred for 16 h. Liquid chromatography - mass spectrometry (LCMS) showed that the starting material was consumed completely. (Desired product RT=0.996 min, 25%; di-$CF_3CO$ byproduct RT=1.125 min, 47%). The reaction mixture was concentrated, and the residue was diluted with 15 ml methanol. The resulted suspension was heated to 70°C and hold for 1 h. Then the reaction mixture was cooled to 15°C. The undissolved solid was collected by filtration. White solid intermediate (346.00 mg, 681.60 umol, with 56.33% yield) was obtained, and was used in next step without further purification.

2) Preparation of compound S1-2. Firstly, the intermediate (130.00 mg, 256.09 umol, 1.00 eq) and $Et_3N$ (182.00 mg, 1.80 mmol, 7.02 eq) were dissolved in 2 ml DCM, and then cooled in ice-bath. Then ethyl isocyanate (isocyanatoethane) (125.00 mg, 1.76 mmol, 6.87 eq) dissolved in 0.5 ml DCM was added. Then the reaction mixture was stirred at 45°C for 48 h. TLC showed that the starting material was consumed completely. The reaction mixture was concentrated under vacuum. The residue was washed with 2.5 ml methanol. White solid product Series 1-2 (30.00 mg, 51.84 umol, 20.24% yield) was obtained through filtration.

3) Detection of the inhibition effect of the compound Series 1-2 on RSV replication. Human respiratory syncytial virus Long strain (ATCC No. VR-26) and HEp-2 cells (ATCC No. CCL-23) were used for infection assays. Cells were maintained in DMEM, supplemented with penicillin/streptomycin and 10% FBS, and incubated at 37°C, 5% $CO_2$. Virus was passaged in HEp-2 cells under the same conditions, except that a concentration of FBS was 2%. Virus stocks were prepared by infecting confluent HEp-2 cells at a low multiplicity of infection (MOI) of 0.1 for two to three days. Cells and virus-containing supernatant were then subjected to a single freeze/thaw cycle at - 80°C to release cell-bound virus, and the sample was clarified by centrifugation at a speed of 2000xg for 10 min at 4°C. The supernatant was homogenized, aliquoted and stored at -20°C.The anti-hRSV potency of the compound was evaluated by focus reduction assay, and the wells containing 50 to 100 focus forming units were selected to count the number of plaques. The compound was tested either during virus adsorption process with compound treatment for 1 h at 4°C to investigate the effect of the compound on the viral entry process, or during post-adsorption process with compound treatment for 72 h at 37°C to investigate the effect of the compound on viral propagation and replication processes, or during all stages of infection. The concentration of the compound resulting in 50% inhibition ($IC_{50}$) of virus replication was determined by focus counting (for viral adsorption process) or focus size measurement (for viral propagation and replication processes) and by using non-linear regression analysis through GraphPad Prism (GraphPad Software, La Jolla California USA). To assess the cytotoxicity of the compound, a CellTiter-Glo Luminescent Cell Viability Assay (Promega, Madison, WI) was carried following the manufacturer's instruction under the conditions of the focus reduction assay.

The detailed protocol is as follows:

Day 1: Seed Hep-2 cells in 24-well plate with a seeding density of $2\times10^5$ cells/well, and incubate cells with 0.5 ml DMEM comprising 10% FBS. Incubate for 16-24 h at 37°C, 5% $CO_2$.

Day 2: Compound treatment and virus infection.

1. compound dilution: the diluent was DMEM comprising 25% DMSO. The compound was diluted to have a concentration of 800 μM and 200 μM;

2. virus dilution: the diluent was DMEM comprising 2% FBS, the hRSV virus stock was hRSV Long-P9 ($2.9\times10^7$ pfu/ml, stored at -80°C). The thawing and the use of the strain were conducted on ice, MOI=0.1.
3. compound treatment and virus infection: pour off the medium in the 24-well plate, rinse the cells once with PBS, add 0.5 ml of the diluted virus, and 5 ml of the diluted compound to make the multiplicity of infection (MOI) to be 0.1, and the final drug concentration was 8 μM, or 2 μM;
4. The plates were incubated at 37°C, 5% $CO_2$ for 3 days, and cytopathic effect was observed.

Day 4: Seed new Hep-2 cells for titration
Seed HEp-2 cells in new 24-well plates with a seeding density of $1.5\times10^5$cells/well. Incubate at 37°C, 5% $CO_2$ for 16-24 h.

Day 5: Collect virus and virus titration

1. Put the 24-well plate in a -80°C freezer for 1h, thaw the plate on ice, and transfer the virus solution in the wells into centrifugal tubes (conducted on ice). Centrifugate at a speed of 8000 rpm for 5 min, and then pipette out 50 μl of the supernatant into a new tube to test virus titer, and the rest virus samples were stored in -80°C freezer.
2. virus dilution: Prepare six EP tubes (1.5ml), mark, and place on ice. Add 270 μl of DMEM medium containing 2% FBS in each tube. Add 30 μl virus solution into the 270 μl DMEM medium, serially dilute to get $10^{-1}$ to $10^{-6}$ virus dilutions.
3. Remove cell culture medium, and rinse cells once with PBS;
4. Infect monolayer HEp-2 cells with 200 μl virus dilutions correspondingly.
5. Incubate for 1.5 h in a 5% $CO_2$ incubator at 37°C. Shake every 15 min.
6. Place the DMEM medium containing 2% FBS and 0.8% CMC in a water bath at 37°C for 1h, then pipette out virus solution after incubation for 1.5 h, and wash cells twice with PBS. Add DMEM comprising 2% FBS and 0.8% CMC per well. Incubate for 3-4 days at 37°C, 5% $CO_2$.

Day 8:

1. Pipette out CMC-containing medium and wash cells once with PBS (conducted on ice);
2. Fix the cells with 4% paraformaldehyde (PFA) for at least 20 min, then wash with PBS, and air dry;
3. Incubate the cells with PBS comprising 0.25% tritonX-100 for 20 minutes. Wash the plate with PBS for 3 times, and each time lasts for 4 min;
4. Incubate the cells for 1 hour at room temperature with 200 μl of primary anti-hRSV F antibody (mouse-Fizgerald, Acton, MA), which was diluted at 1:1000 in PBS containing 5% skim milk;
5. Wash for three times with PBS containing 0.02% Tween 20, and each time lasts for 4 min;
6. Add 200 μl of a secondary HRP-conjugated antibody (goat antimouse-Bethyl, Montgomery, TX) into the wells, the antibody was diluted at 1:6000 in PBS containing 5% skim milk; and incubate at room temperature for 1 h.
7. Wash the monolayers with PBS containing 0.02% Tween 20 for 3 times, and each time lasts for 4 min
8. Add 200 μl of True Blue substrate into the plate, and leave at room temperature in the dark for more than 10 min and till blue spots appear;
9. Rinse with running water;
10. Dry in oven, scan plate, and count blue plaques;

As seen in FIG. 4, the maximum inhibition effect of the compound S1-2 on RSV replication is 99% (compared with the blank control group.).

4) Detection of the inhibition effect of the compounds on Hh signaling pathway. NIH3T3 cells were seeded in a 6-well plate ($3\times10^5$ cell/well), or a 12-well plate ($1.5\times10^5$ cell/well) one day in advance, cultivated with DMEM comprising 5% FBS without antibiotics per well to ensure 90-95% confluence at the time of transfection. Diluted plasmids were mixed with Lipofectamine 2000 (a total volume is 100-1000 μl), and were added to each well containing cells and medium, then the plate was incubated in a $CO_2$ incubator at 37°C for 48 h. After transfection for 48 h, SAG was added to reach a final concentration of 0.5 μM. The compounds of various concentrations were added. After incubation with the compound for 48 h, the cells were washed, lysed, and the lysate transferred to a 96-well white plate. LAR II reagent (Promega, Dual-luciferase reporter assay system, E1910) was added, and the data of the samples were read in a luminescence reader.

The detailed protocol was as follows:

Day 1: NIH3T3 cells were seeded in a 12-well plate (1.5×$10^5$ cell/well) one day in advance, cultivated with DMEM containing 5% FBS without antibiotics per well so as to ensure 90-95% confluence at the time of transfection.

Day 2: For each transfection sample, the DNA-Lipofectamine 2000 complexes were prepared as follows: 1. Dilute DNA (the DNA comprising Glibinding site-luciferase expressing plasmid and Renilla luciferase expressing plasmid mixed in a ratio of 50:1) with 250 μl of Opti-MEM. Mix gently.

2. Mix Lipofectamine 2000 gently before use, then dilute with 250 μl of Opti-MEM in new centrifugal tubes. Mix gently.

3. After incubation Lipofectamine 2000 for between 5 and 30 min, combine the diluted DNA with the diluted Lipofectamine 2000. Mix gently and incubate for 20 min at room temperature to allow the formation of stable DNA-Lipofectamine 2000 complexes.

4. Add the DNA-Lipofectamine 2000 complexes to each well containing cells and medium. Mix gently by rocking the plate back and forth.

5. After 4-6 h, pipette out the medium from the well, and add growth medium. Then incubate the cells in an incubator (37°C, 5% $CO_2$) for 48 h.

Day 4: After transfection for 48h, diluent the drug with DMEM comprising 0.5% FBS and 0.25% DMSO. SAG was diluted to have a concentration of 1 μM, and CPM and the compounds were diluted to have a concentration of 20 μM. 250 μl of SAG with a concentration of 1 μM and 250 μl of CPM or the compounds with a concentration of 20 μM were added to the same well. The final concentration of SAG is 0.5 μM, and the final concentration of CPM or the compounds is 10 μM,

Day 5:

1. Remove the growth media for the cultured cells. Rinse cultured cells with PBS. Remove the rinse solution.
2. Lyse the cells according to the operating manual of the Dual-luciferase reporter assay kit (Promega, E1910) and dispense 100 μl of a lysing agent into each culture vessel. Gently rock/shake the culture vessel for 15 min at room temperature.
3. Add 20 μl of the cell lysate to a 96-well plate, and add 50 μl of LAR II. Read the plate and measure the firefly luciferase activity.
4. Add 50 μl of stop&Glo reagent. Read the plate and measure the Renilla luciferase activity.

Record all the data and calculate the ratio. All the luminescence data were deducted with blank data. The firefly luciferase activity datum in the group with SAG and with DMSO control was seen as 1, and the data in other groups were normalized. The obtained ratios were labeled as "ratio 1". Then, the Renilla luciferase activity datum in the group with SAG and with DMSO control was seen as 1, and the data in other groups were normalized. The obtained ratios were labeled as "ratio 2". Finally, the ratio of "ratio 1" to "ratio 2" was calculated, and the ratio was the normalized expression level (percentage) of the firefly luciferase.

As seen from FIG. 5, the compound S1-2 shows unsignificant inhibition effect on gene expression, with the inhibition percentage being 36%. The data indicated that anti-RSV effect of the steroid alkaloids derived from CPM is independent from their inhibition effect on the Hh signaling pathway.

5) Calculate the coefficient of inhibition (ICrh). As seen in FIG. 6, the ICrh value of S1-2 is 1.44596, which is larger than 1, indicating that the compound exhibits beneficial inhibition property.

6) Screening the compound S1-2. As seen in FIG. 6, the compound S1-2 has beneficial inhibition property, and can be used to treat RSV infection.

Example 2

The preparation process of the compound S1-4 (FIGS. 1-3) was as follows:

1) Preparation of the intermediate. The procedure was the same as that in example 1.
2) Preparation of the compound S1-4. The procedure was the same as that in example 1.
3) Detection of the inhibition effect of S1-4 on RSV replication. The detection process was the same as that in example 1. As seen in FIG. 4, the maximum inhibition percentage of S1-4 on RSV replication is 88%.
4) Detection of the inhibition effect of S1-4 on Hh signaling pathway. The detection process was the same as that

in example 1. As seen in FIG. 5, the inhibition effect of the compound S1-4 on gene expression is not significant, with the inhibition percentage being 5%. The data indicated that, the anti-RSV effect of the steroid alkaloids derived from CPM was independent from their inhibition effect on the Hh signaling pathway.

5) Calculate the coefficient of inhibition (ICrh). As seen in FIG. 6, the ICrh value of S1-4 was 1.6172823, and was larger than 1, indicating that the compound exhibits beneficial inhibition property.

6) Screening the compound S1-4. As shown in FIG. 6, the compound S1-4 has beneficial inhibition property. Furthermore, S1-4 has the largest ICrh value among the tested compounds, indicating that the compound can be used to treat RSV infection.

## Claims

1. A compound for use in the treatment of respiratory syncytial virus infection, **characterized in that** the compound is S1-2 of formula (III) or S1-4 of formula (IV):

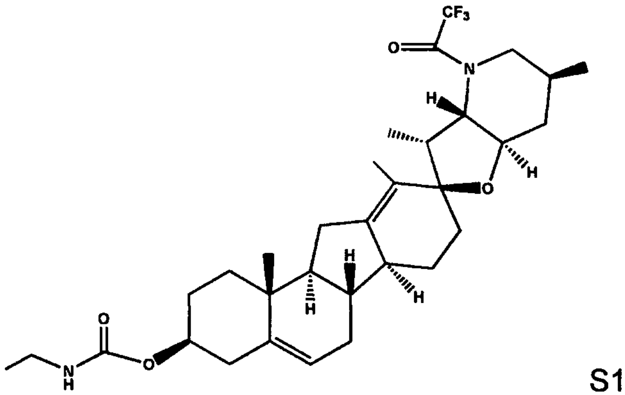

S1-2,

(III)

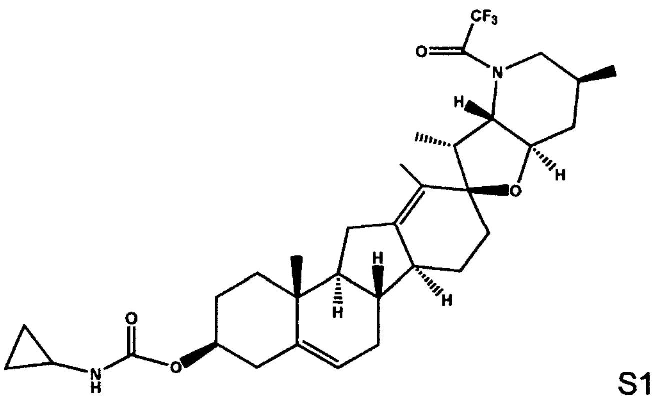

S1-4.

(IV)

2. A method of synthesizing the compound according to claim 1, **characterized in that** the method comprises:

1) dissolving cyclopamine and triethylamine in dry dichloromethane (DCM);
2) adding trifluoroacetic acid anhydride dissolved in DCM into the solution under 0°C;
3) heating the reaction mixture slowly to a temperature of 15°C and stirring for 16 h;
4) confirming the consumption of the initial raw materials by liquid chromatography-mass spectrometry;
5) concentrating the reaction mixture and diluting the residues with methanol;
6) heating the obtained suspension to 70°C and holding for 1 h;
7) cooling the reaction mixture to 15°C;
8) filtrating to obtain white solid intermediates;
9) dissolving the intermediates and triethylamine in dry dichloromethane (DCM), and cooling in ice;

10) adding isocyanato ethane dissolved in DCM into the solution;
11) stirring the reaction mixture at 45°C for 48 h;
12) confirming that the initial raw materials are consumed completely by thin layer chromatography (TLC);
13) concentrating the reaction mixture in vacuum;
14) cleaning the residues with methanol; and
15) filtrating to obtain white solid compound of claim 1.

**3.** A method of testing the inhibitory effect of the compound according to claim 1 on the replication of respiratory syncytial virus, **characterized in that** the method comprises the following steps:

1) human respiratory syncytial virus Long strain and HEp-2 cells are used for infection assays;
2) cells are maintained in DMEM, supplemented with penicillin/streptomycin and 10% fetal bovine serum (FBS), and incubated at 37 C, 5% $CO_2$;
3) virus is passaged in HEp-2 cells under the same conditions, except that a concentration of FBS is 2%;
4) virus stocks are prepared by infecting confluent HEp-2 cells at a low multiplicity of infection (MOI) of 0.1 for two to three days; cells and virus-containing supernatant are then subjected to a single freeze/thaw cycle at -80°C to release cell-bound virus;
5) the sample is clarified by centrifugation at a speed of 2000xg for 10 min at 4°C, the supernatant is homogenized, aliquoted and stored at -20°C;
6) the anti-hRSV potency of the compound is evaluated by focus reduction assay, and the wells containing 50 to 100 focus forming units are selected to count the number of plaques; the compound is tested either during virus adsorption process with compound treatment for 1 h at 4°C to investigate the effect of the compound on the viral entry process, or during post-adsorption process with compound treatment for 72 h at 37°C to investigate the effect of the compound on viral propagation and replication processes, or during all stages of infection; the concentration of compound resulting in 50% inhibition ($IC_{50}$) of virus replication is determined by focus counting or focus size measurement and by using non-linear regression analysis through GraphPad Prism software; and
7) a CellTiter-Glo Luminescent Cell Viability Assay is carried following the manufacturer's instruction under conditions of the focus reduction assay to assess the cytotoxicity of the compound.

**4.** A method of testing the inhibitory effect of the compound according to claim 1 on the Hh signaling pathway, **characterized in that** the method comprises the following process:

1) NIH3T3 cells are seeded in a 6-well plate, or a 12-well plate one day in advance, cultivated with DMEM comprising 5% FBS without antibiotics so as to ensure 90~95% confluence at the time of transfection;
2) diluted plasmids are mixed with Lipofectamine 2000, cultivated and are added to each well containing cells and medium, then the plate is incubated in a $CO_2$ incubator at 37°C for 48 h
3) after transfection for 48 h, SAG is added until a final concentration is 0.5 μM, and CPM analogues of various concentrations are added;
4) at the end of the incubation, cells are washed, lysed, and the lysate is transferred to a 96-well white plate; and
5) LAR II reagent is added, and the data for the samples are read in a luminescence reader; the compound in the RSV inhibition assay is tested in the Hh signaling pathway inhibition assay, in which the inhibition effect of the analogues on SAG induced and Smo-dependent gene expression is tested.

**5.** The compound for use according to claim 1, **characterized in that** the compound is used in the treatment of respiratory syncytial virus infection, capillary bronchitis caused by respiratory syncytial virus, pneumonia caused by respiratory syncytial virus, or tympanitis caused by respiratory syncytial virus.

## Patentansprüche

**1.** Verbindung zur Verwendung bei der Behandlung einer respiratorisches Synzytial-Virus-Infektion, **dadurch gekennzeichnet, dass** die Verbindung S1-2 der Formel (III) oder S1-4 der Formel (IV) ist:

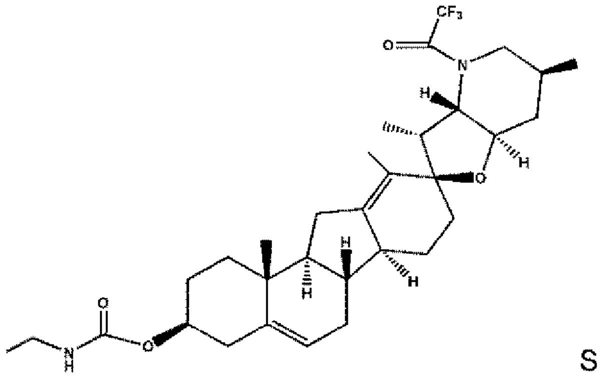

S1-2,

(III)

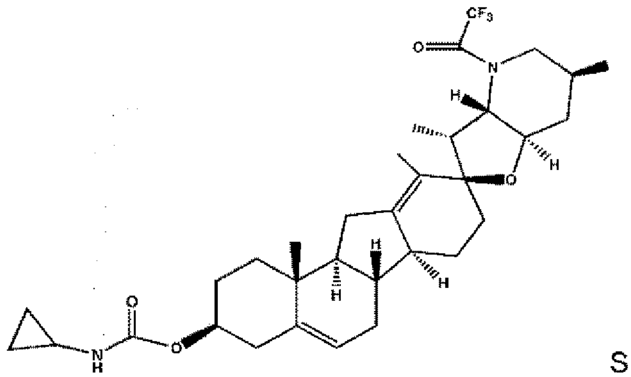

S1-4.

(IV)

**2.** Verfahren zum Synthetisieren der Verbindung nach Anspruch 1, **dadurch gekennzeichnet, dass** das Verfahren umfasst:

1) Auflösen von Cyclopamin und Triethylamin in trockenem Dichlormethan (DCM);
2) Zugabe von in DCM gelöstem Trifluoressigsäureanhydrid zu der Lösung bei 0 °C;
3) langsames Erhitzen der Reaktionsmischung auf eine Temperatur von 15 °C und Rühren für 16 h;
4) Bestätigung des Verbrauchs der anfänglichen Rohmaterialien durch Flüssigchromatographie-Massenspektrometrie;
5) Konzentrieren der Reaktionsmischung und Verdünnen der Rückstände mit Methanol;
6) Erhitzen der erhaltenen Suspension auf 70 °C und Halten für 1 h;
7) Abkühlen der Reaktionsmischung auf 15 °C;
8) Filtrieren, um weiße feste Zwischenprodukte zu erhalten;
9) Auflösen der Zwischenprodukte und Triethylamin in trockenem Dichlormethan (DCM) und Kühlen in Eis;
10) Zugabe von in DCM gelöstem Isocyanatoethan zu der Lösung;
11) Rühren der Reaktionsmischung bei 45 °C für 48 h;
12) Bestätigung, dass die anfänglichen Rohmaterialien durch Dünnschichtchromatographie (TLC) vollständig verbraucht wurden;
13) Konzentrieren der Reaktionsmischung im Vakuum;
14) Reinigen der Rückstände mit Methanol; und
15) Filtrieren, um eine weiße feste Verbindung nach Anspruch 1 zu erhalten.

**3.** Verfahren zum Testen der hemmenden Wirkung der Verbindung nach Anspruch 1 auf die Replikation des respiratorischen Synzytial-Virus, **dadurch gekennzeichnet, dass** das Verfahren die folgenden Schritte umfasst:

1) humane respiratorische Synzytial-Virus-Langstamm- und HEp-2-Zellen werden für Infektionsassays verwendet;

2) Zellen werden in DMEM, ergänzt mit Penicillin/Streptomycin und 10 % fötalem Rinderserum (FBS), gehalten und bei 37 °C, 5 % $CO_2$ inkubiert;
3) Virus wird in HEp-2-Zellen unter den gleichen Bedingungen passagiert, außer dass die Konzentration von FBS 2 % beträgt;
4) Virusvorräte werden hergestellt, indem konfluente HEp-2-Zellen bei einer niedrigen Infektionsmultiplizität (MOI) von 0,1 für zwei bis drei Tage infiziert werden; Zellen und virushaltiger Überstand werden dann einem einzigen Einfrier/Auftau-Zyklus bei -80 °C unterzogen, um zellgebundenes Virus freizusetzen;
5) die Probe wird durch Zentrifugation bei einer Geschwindigkeit von 2000 x g für 10 min bei 4 °C geklärt, der Überstand wird homogenisiert, aliquotiert und bei -20 °C gelagert;
6) die Anti-hRSV-Potenz der Verbindung wird durch einen Fokusreduktionsassay bewertet, und die Vertiefungen, die 50 bis 100 fokusbildende Einheiten enthalten, werden ausgewählt, um die Anzahl der Plaques zu zählen; die Verbindung wird entweder während des Virusadsorptionsprozesses mit Verbindungsbehandlung für 1 h bei 4 °C getestet, um die Wirkung der Verbindung auf den viralen Eintrittsprozess zu untersuchen, oder während des Postadsorptionsprozesses mit Verbindungsbehandlung für 72 h bei 37 °C, um die Wirkung der Verbindung auf virale Ausbreitungs- und Replikationsprozesse zu untersuchen, oder während aller Stadien der Infektion getestet; die Konzentration der Verbindung, die zu einer 50 %-igen Hemmung ($IC_{50}$) der Virusreplikation führt, wird durch Fokuszählung oder Fokusgrößenmessung und durch Verwendung nichtlinearer Regressionsanalyse durch die GraphPad Prism-Software bestimmt; und
7) ein CellTiter-Glo Luminescent Cell Viability Assay wird gemäß den Anweisungen des Herstellers unter den Bedingungen des Fokusreduktionsassays durchgeführt, um die Zytotoxizität der Verbindung zu bewerten.

**4.** Verfahren zum Testen der inhibitorischen Wirkung der Verbindung nach Anspruch 1 auf den Hh-Signalweg, **dadurch gekennzeichnet, dass** das Verfahren den folgenden Prozess umfasst:

1) NIH3T3-Zellen werden einen Tag im Voraus in eine Platte mit 6 Vertiefungen oder eine Platte mit 12 Vertiefungen ausgesät und mit DMEM kultiviert, das 5 % FBS ohne Antibiotika umfasst, um eine Konfluenz von 90~95 % zum Zeitpunkt der Transfektion sicherzustellen;
2) verdünnte Plasmide werden mit Lipofectamin 2000 gemischt, kultiviert und in jede Vertiefung gegeben, die Zellen und Medium enthält, dann wird die Platte in einem $CO_2$-Inkubator bei 37 °C für 48 h inkubiert
3) nach Transfektion für 48 h wird SAG zugegeben, bis eine Endkonzentration von 0,5 $\mu$M beträgt, und CPM-Analoga verschiedener Konzentrationen werden zugegeben;
4) am Ende der Inkubation werden die Zellen gewaschen, lysiert und das Lysat wird auf eine weiße Platte mit 96 Vertiefungen übertragen; und
5) LAR II-Reagenz wird hinzugefügt und die Daten für die Proben werden in einem Lumineszenzlesegerät abgelesen; die Verbindung im RSV-Inhibitionsassay wird im Hh-Signalweg-Inhibitionsassay getestet, bei dem die Hemmwirkung der Analoga auf die SAG-induzierte und Smo-abhängige Genexpression getestet wird.

**5.** Verbindung zur Verwendung nach Anspruch 1, **dadurch gekennzeichnet, dass** die Verbindung zur Behandlung einer respiratorischen Synzytiai-Virus-Infektion, einer durch ein respiratorisches Synzytial-Virus verursachten Kapillarbronchitis, einer durch ein respiratorisches Synzytial-Virus verursachten Pneumonie oder einer durch ein respiratorisches Synzytial-Virus verursachten Tympanitis verwendet wird.

## Revendications

**1.** Composé destiné à être utilisé dans le traitement d'une infection par le virus syncytial respiratoire, **caractérisé en ce que** le composé est S1-2 de formule (III) ou S1-4 de formule (IV) :

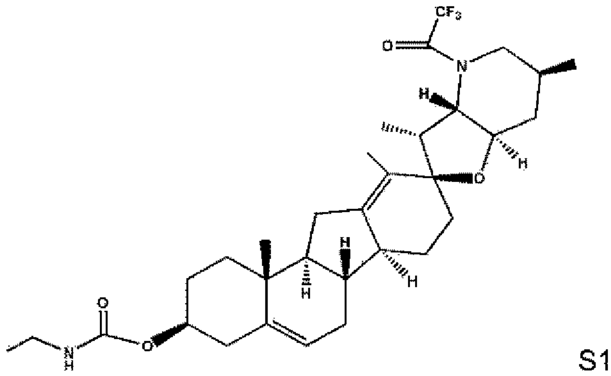

S1-2,

(III)

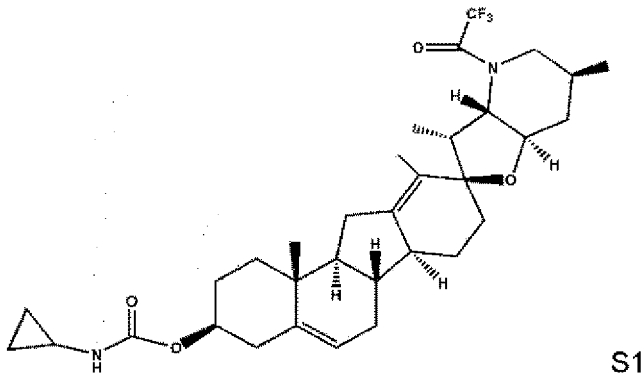

S1-4.

(IV)

**2.** Procédé de synthèse du composé selon la revendication 1, **caractérisé en ce que** le procédé comprend les étapes consistant à :

1) dissoudre de la cyclopamine et de la triéthylamine dans du dichlorométhane anhydre (DCM) ;
2) ajouter de l'anhydride d'acide trifluoroacétique dissous dans du DCM dans la solution sous 0 °C ;
3) chauffer le mélange réactionnel lentement à une température de 15 °C et agiter durant 16 h ;
4) confirmer la consommation des matières brutes initiales par chromatographie liquide-spectrométrie de masse ;
5) concentrer le mélange réactionnel et diluer les résidus avec du méthanol ;
6) chauffer la suspension obtenue à 70 °C et maintenir durant 1 h ;
7) refroidir le mélange réactionnel à 15 °C ;
8) filtrer pour obtenir des intermédiaires solides blancs ;
9) dissoudre les intermédiaires et la triéthylamine dans du dichlorométhane anhydre (DCM) et refroidir dans de la glace ;
10) ajouter du isocyanato éthane dissous dans du DCM dans la solution ;
11) agiter le mélange réactionnel à 45 °C durant 48 h ;
12) confirmer que les matières brutes initiales sont consommées complètement par chromatographie sur couche mince (TLC) ;
13) concentrer le mélange réactionnel sous vide ;
14) nettoyer les résidus avec du méthanol ; et
15) filtrer pour obtenir un composé solide blanc selon la revendication 1.

**3.** Procédé de test de l'effet inhibiteur du composé selon la revendication 1 sur la réplication du virus syncytial respiratoire, **caractérisé en ce que** le procédé comprend les étapes suivantes :

1) la souche Longue du virus syncytial respiratoire humain et des cellules HEp-2 sont utilisées pour les tests

d'infection ;
2) les cellules sont maintenues dans du DMEM, complémentées avec de la pénicilline/streptomycine et 10 % de sérum bovin fœtal (FBS) et incubées à 37 °C, $CO_2$ à 5 % ;
3) le virus est passé dans des cellules HEp-2 dans les mêmes conditions, si ce n'est qu'une concentration de FBS est de 2 % ;
4) des stocks de virus sont préparés en infectant des cellules HEp-2 confluentes à une faible multiplicité d'infection (MOI) de 0,1 durant deux à trois jours ; les cellules et le surnageant contenant le virus sont ensuite soumis à un seul cycle de congélation/décongélation à -80 °C pour libérer le virus lié aux cellules ;
5) l'échantillon est clarifié par centrifugation à une vitesse de 2000×g durant 10 minutes à 4 °C, le surnageant est homogénéisé, aliquoté et stocké à -20 °C ;
6) la puissance anti-hRSV du composé est évaluée par un test de réduction de foyer, et les puits contenant 50 à 100 unités formant foyer sont choisis pour compter le nombre de plaques ; le composé est testé soit durant le processus d'adsorption du virus avec le traitement du composé durant 1 h à 4 °C pour examiner l'effet du composé sur le processus d'entrée virale, soit durant le processus de post-adsorption avec le traitement du composé durant 72 h à 37 °C pour examiner l'effet du composé sur les processus de propagation et de réplication virales soit durant tous les stades d'infection ; la concentration du composé résultant dans 50 % d'inhibition ($CI_{50}$) de réplication du virus est déterminée par comptage de foyers ou mesure de la taille du foyer et en utilisant une analyse de régression non linéaire par le logiciel GraphPad Prism ; et
7) un test de viabilité de cellule luminescente CellTiter-Glo est réalisé suivant les instructions du fabricant dans des conditions de test de réduction de foyer pour évaluer la cytotoxicité du composé.

4. Procédé de test de l'effet inhibiteur du composé selon la revendication 1 sur la voie de signalisation Hh, **caractérisé en ce que** le procédé comprend le processus suivant :

1) des cellules NIH3T3 sont ensemencées dans une plaque à 6 puits, ou une plaque à douze puits un jour à l'avance, cultivées avec du DMEM comprenant 5 % de FBS sans antibiotiques pour assurer 90 ~ 95 % de confluence au moment de la transfection ;
2) les plasmides dilués sont mélangés avec de la lipofectamine 2000, cultivés et sont ajoutés à chaque puits contenant des cellules et du milieu, puis la plaque est incubée dans un incubateur à $CO_2$ à 37 °C durant 48 h ;
3) après la transfection durant 48 h, du SAG est ajouté jusqu'à une concentration finale de 0,5 $\mu$M, et des analogues de CPM de diverses concentrations sont ajoutés ;
4) à la fin de l'incubation, les cellules sont lavées, lysées et le lysat est transféré dans une plaque blanche à 96 puits ; et
5) un réactif LAR II est ajouté et les données pour les échantillons sont lues dans un lecteur de luminescence ; le composé dans le test d'inhibition de RSV est testé dans le test d'inhibition de la voie de signalisation Hh, dans lequel l'effet d'inhibition des analogues sur l'expression génique induite par SAG et dépendante de Smo est testé.

5. Composé destiné à être utilisé selon la revendication 1, **caractérisé en ce que** le composé est utilisé dans le traitement d'une infection par le virus syncytial respiratoire, d'une bronchite capillaire provoquée par le virus syncytial respiratoire, d'une pneumonie provoquée par le virus syncytial respiratoire ou d'une tympanite provoquée par le virus syncytial respiratoire.

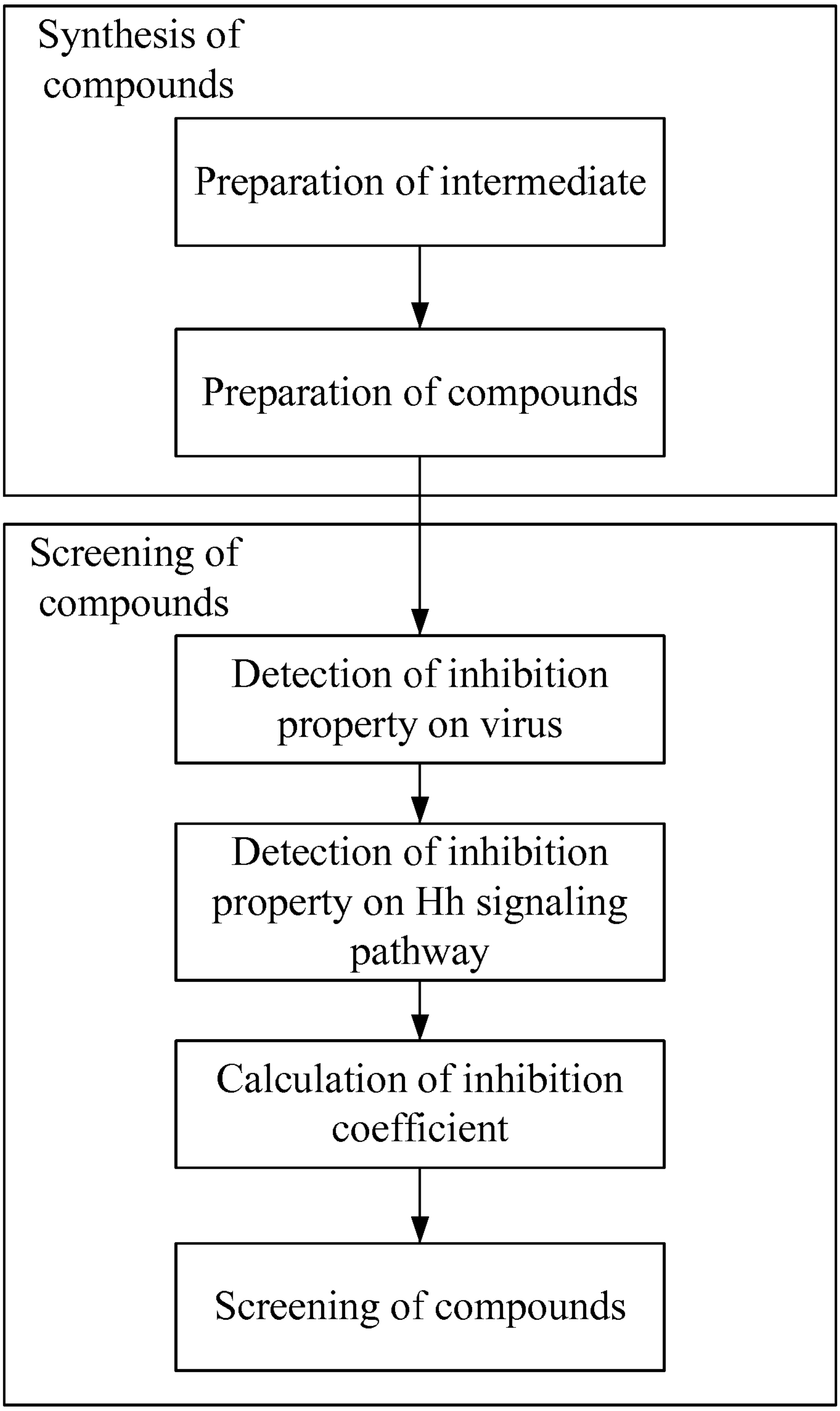
Synthesis of compounds
Preparation of intermediate
Preparation of compounds
Screening of compounds
Detection of inhibition property on virus
Detection of inhibition property on Hh signaling pathway
Calculation of inhibition coefficient
Screening of compounds

FIG. 1

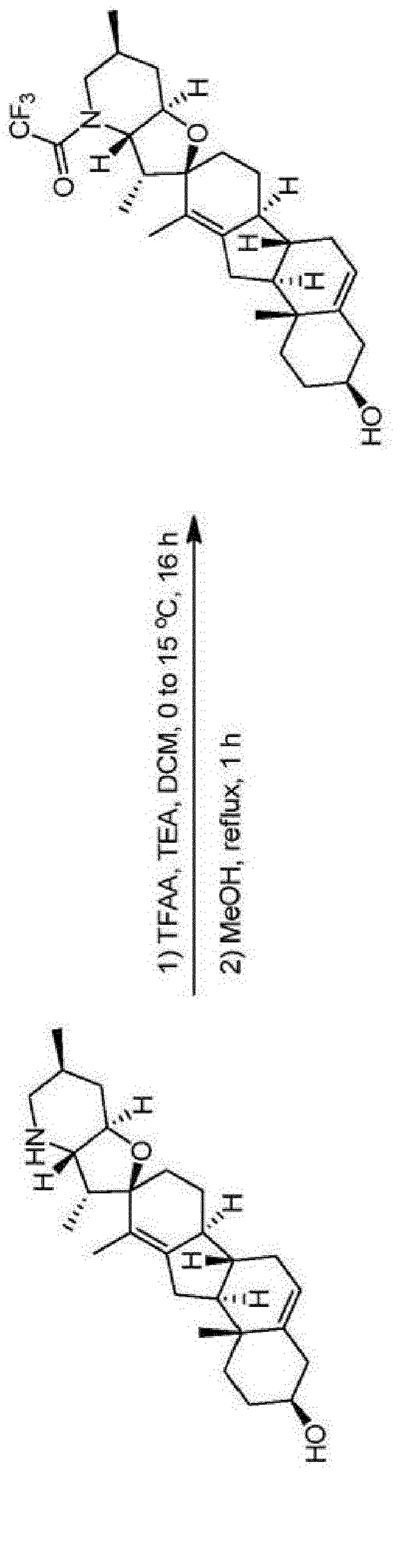


Cyclopamine

Intermediate

FIG. 2

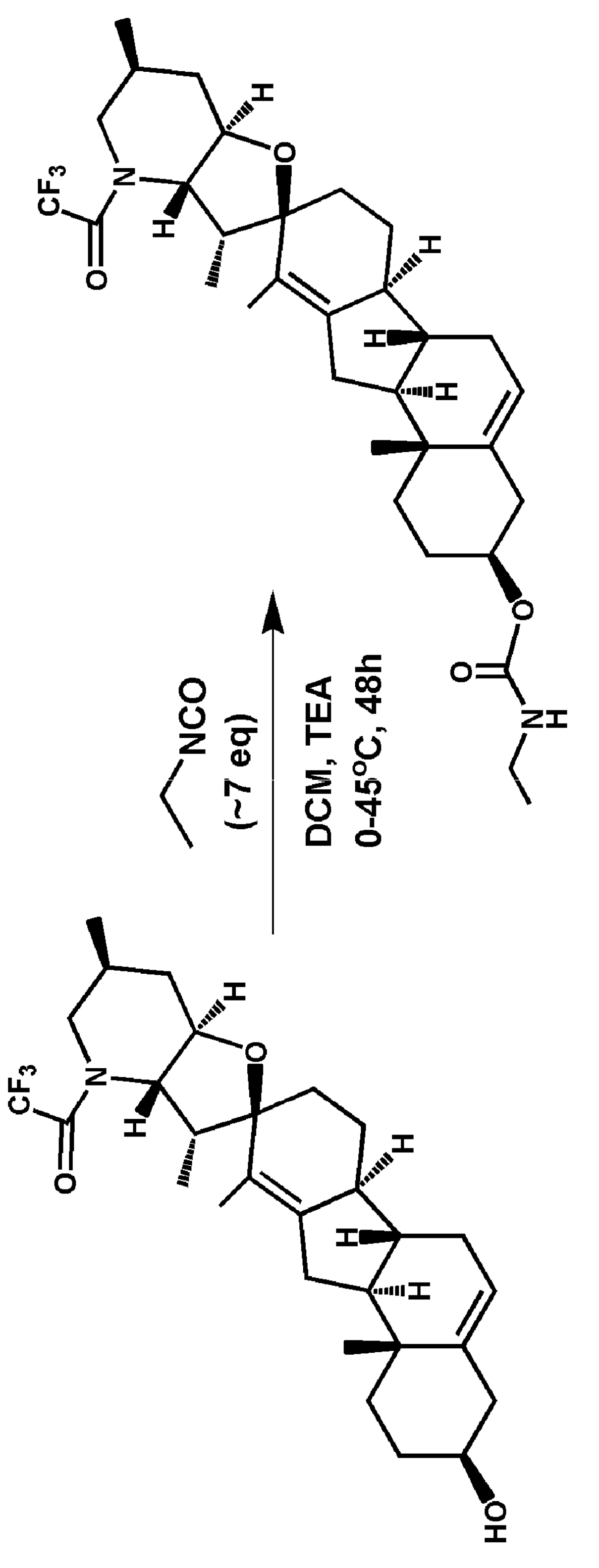


Intermediate

Series 1-2

FIG. 3

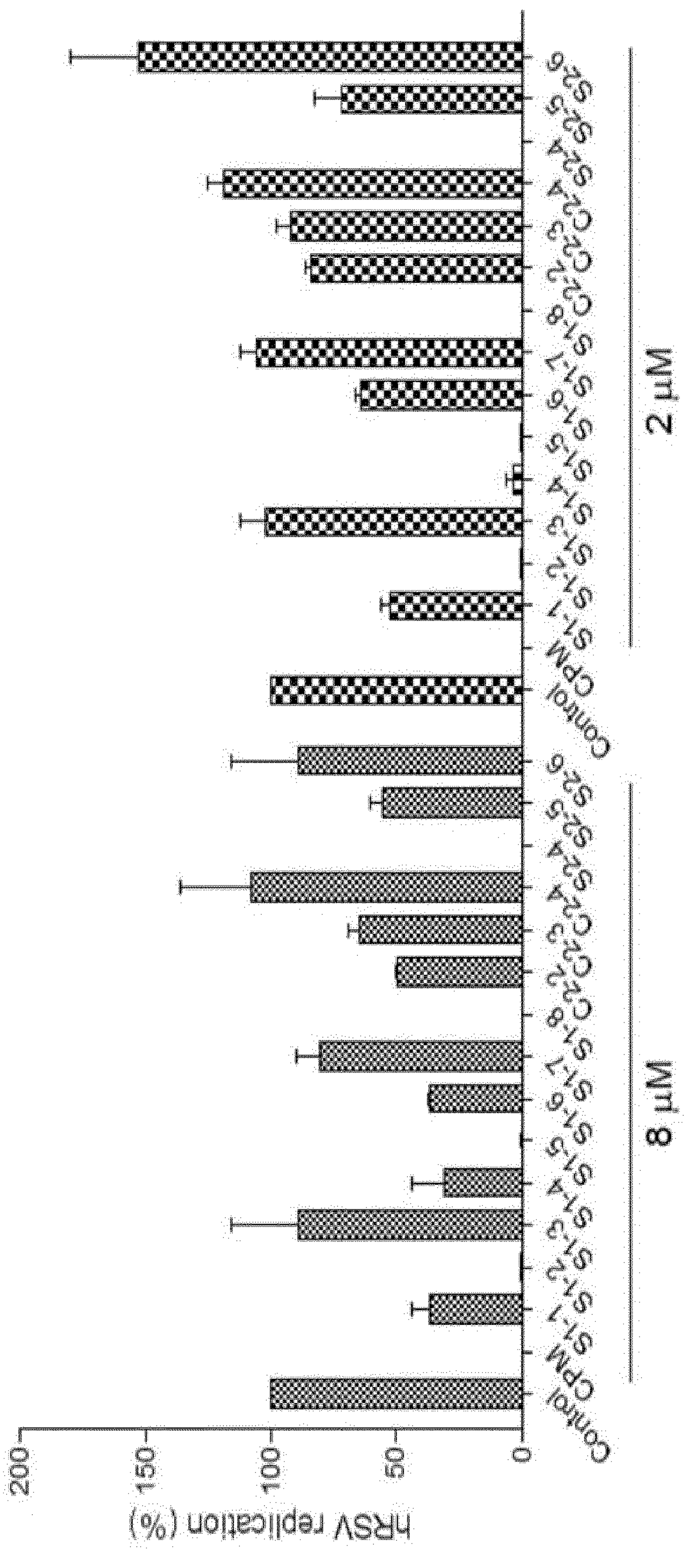
hRSV replication (%)
0
50
100
150
200
Control
CPM
S1-1
S1-2
S1-3
S1-4
S1-5
S1-6
S1-7
S1-8
C2-2
C2-3
C2-4
S2-4
S2-5
S2-6
8 µM
2 µM

FIG. 4

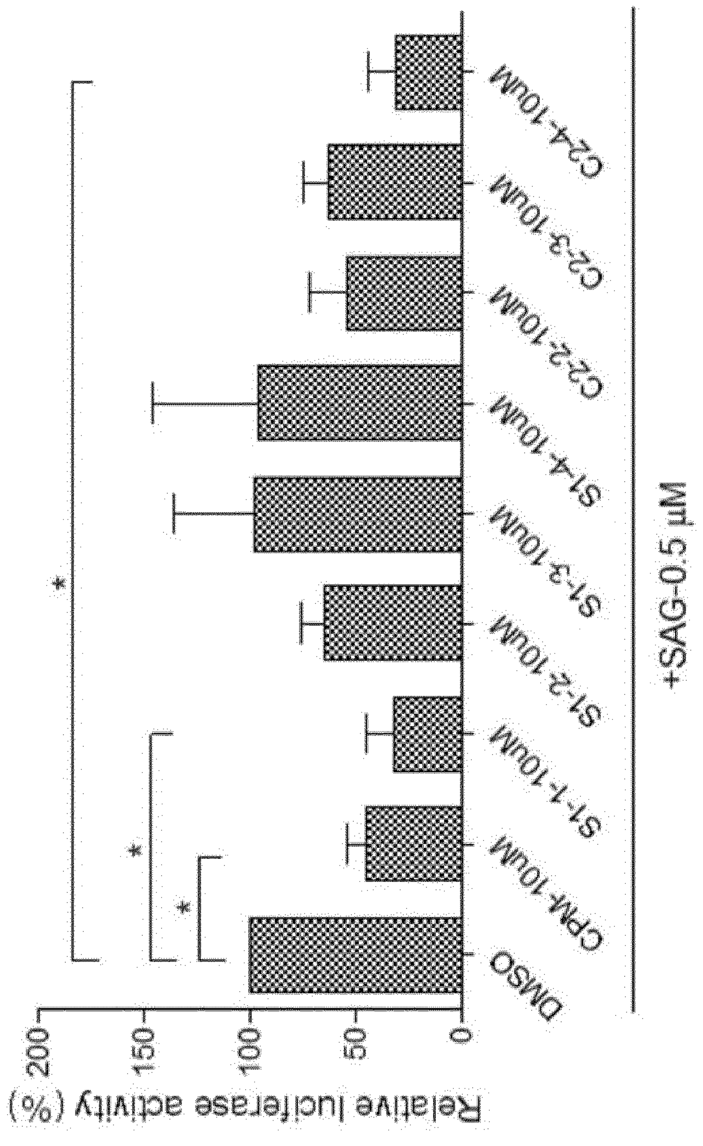
Relative luciferase activity (%)
200
150
100
50
0
*
*
*
DMSO
CPM-10uM
S1-1-10uM
S1-2-10uM
S1-3-10uM
S1-4-10uM
C2-2-10uM
C2-3-10uM
C2-4-10uM
+SAG-0.5 μM

FIG. 5

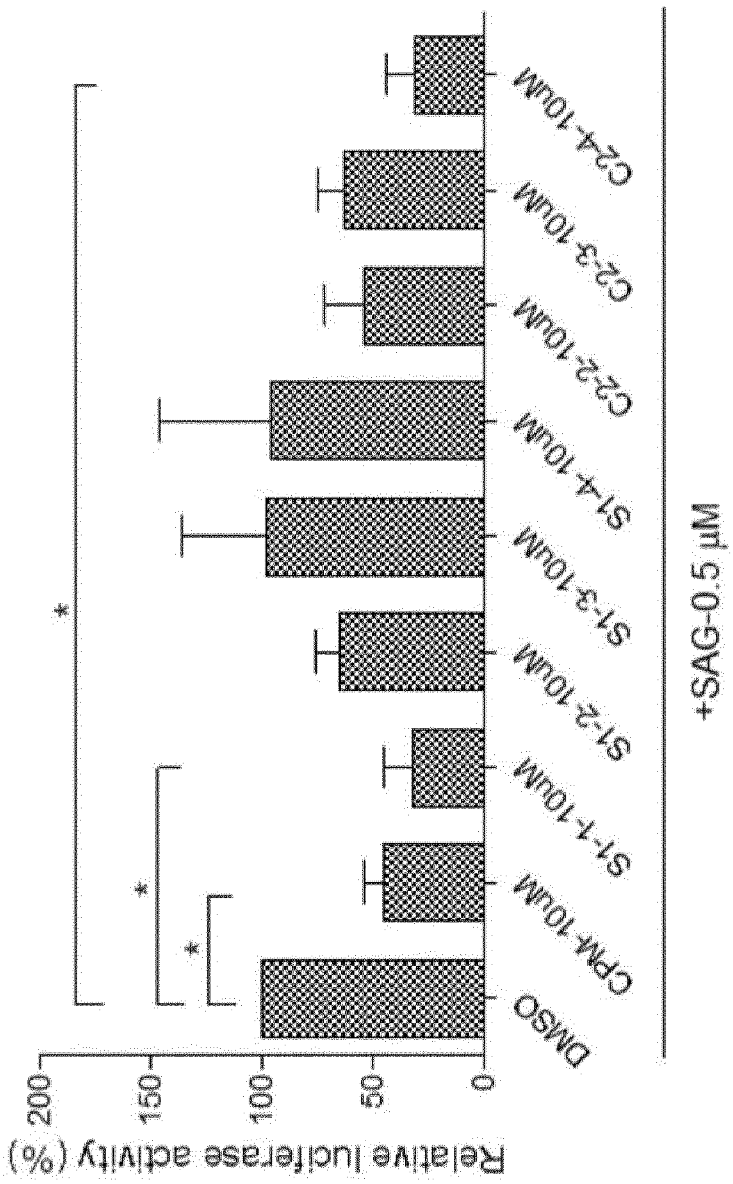
Relative luciferase activity (%)
200
150
100
50
0
*
*
*
DMSO
CPM-10uM
S1-1-10uM
S1-2-10uM
S1-3-10uM
S1-4-10uM
C2-2-10uM
C2-3-10uM
C2-4-10uM
+SAG-0.5 μM

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105232562